# EUROPEAN PATENT APPLICATION

(11) **EP 4 826 732 A1**
(43) Date of publication of application: **21.10.2026**
(21) Application number: 26166461.9
(22) Date of filing: 20.03.2026
(51) Int. Cl.: G16H 20/40, A61B 1/267, A61M 16/01, G16H 30/40, G16H 40/63

(54) **INTEGRATED ANESTHESIA AND VIDEO LARYNGOSCOPY SYSTEM**

(30) Priority: 14.04.2025 US 202519178715
(71) Applicant: GE Precision Healthcare LLC, Waukesha, WI 53188 (US)
(72) Inventor: R MALAGI, Harsha, 560066 Bengaluru (IN); CHRISTOPHER SELVI, Godwin, 560066 Bengaluru (IN); M, Kathiresan, 560066 Bengaluru (IN); ARUL SIMON, Shajin, 560066 Bengaluru (IN); KOTTADAMANE, Sharath, 560066 Bengaluru (IN); RAO R, Ramakrishna, 560066 Bengaluru (IN); VITAGLIANO, Andrea, Madison, 53718 (US); DEAN BAUM, Bradley, Madison, 53718 (US)
(74) Representative: Kilburn & Strode LLP

(57) **Abstract**

Systems are provided for an intubation device (102, 600) that integrates an anesthesia system (104) and a video laryngoscopy system (140). The anesthesia system (104) is configured as to deliver anesthesia to a subject, and the video laryngoscopy system (140) is configured to capture a laryngoscopy video of the subject. The anesthesia system (104) further includes a communication core (106) configured to send and receive data to and from a third-party source (110). The intubation device (102, 600) may generate intubation guidance from data generated and/or captured by the anesthesia system (104) and the video laryngoscopy system (140), and from data received from the third-party source (110); and render, and output for display on a display (128) of the intubation device (102, 600), the data received from the third-party source (110), the data generated and/or captured by the anesthesia system (104) and the video laryngoscopy system (140), and the intubation guidance at the same time.

## Description

### TECHNICAL FIELD

Embodiments of the subject matter disclosed herein relate to systems for an assisted intubation system that integrates insights from additional patient monitoring systems, including anesthesia systems, video laryngoscopy systems, imaging systems, and pre-operative insights.

### BACKGROUND

Certain medical procedures, such as surgery, may include various sub-procedures to prepare a patient for surgery, maintain the patient in a certain condition during surgery (e.g., anesthetized), and help the patient recover after surgery. Such sub-procedures that are performed in support of a main procedure may be referred to as perioperative care. Perioperative care may include administering anesthesia to a patient, and intubating the patient. Video laryngoscopy (VL) is an established method to assist during intubation of a patient, especially in patients with challenging airways and/or positions. VL may provide insights regarding a patient airway via a VL stream.

### BRIEF DESCRIPTION

In one embodiment, an intubation device comprises an anesthesia system configured to deliver anesthesia to a subject (e.g., a patient) and a video laryngoscopy (VL) system configured to capture a laryngoscopy video of the subject. The anesthesia system may comprise a communication core configured to send and receive data to and from a third-party source. The intubation device may further include a display, a processor, and a memory storing computer-readable instructions. The computer-readable instructions may be executed by the processor to generate intubation guidance from data generated and/or captured by the anesthesia system and the VL system, and data received from the third-party source. Further, the processor may render data from the third-party source, data generated and/or captured by the anesthesia system and the VL system, and intubation guidance at the same time on the display.

It should be understood that the brief description above is provided to introduce in simplified form a selection of concepts that are further described in the detailed description. It is not meant to identify key or essential features of the claimed subject matter, the scope of which is defined uniquely by the claims that follow the detailed description. Furthermore, the claimed subject matter is not limited to implementations that solve any disadvantages noted above or in any part of this disclosure.

### BRIEF DESCRIPTION OF THE DRAWINGS

The present invention will be better understood from reading the following description of non-limiting embodiments, with reference to the attached drawings, herein below:
FIG. 1 shows a block diagram of an intubation device including an anesthesia system and a video laryngoscopy system;
FIG. 2 shows a block diagram of the intubation device integrated in a first example communications network;
FIG. 3 shows a block diagram of the intubation device integrated in a second example communications network;
FIG. 4 shows a block diagram of the intubation device integrated in a third example communications network;
FIG. 5 shows a block diagram of the intubation device integrated in a fourth example communications network;
FIG. 6 shows a perspective view of an example of the intubation device;
FIG. 7 shows example display configurations of the intubation device;
FIG. 8 shows an example system architecture of the intubation device;
FIG. 9 shows a high level method for anesthesia phase detection during intubation workflow;
FIG. 10 shows examples of a chest X-ray, a labeled chest X-ray, and patient insights generated from the labeled chest X-ray;
FIG. 11 shows example intubation shapes;
FIG. 12 shows use of an additional imaging device used alongside the intubation system to perform intubation;
FIG. 13 shows a method for acquiring and generating patient insights to assist with intubation;
FIG. 14 shows a first example display view including patient insights;
FIG. 15 shows a second example display view including patient insights;
FIG. 16 shows a third example display view including patient insights;
FIG. 17 shows a fourth example display view including patient insights; and
FIG. 18 shows a high level method for using patient insights generated by the intubation device to assist with intubation.

### DETAILED DESCRIPTION

A conventional video laryngoscope used to perform video laryngoscopy (VL) may be a separate device from an anesthesia device used to administer anesthesia. The video laryngoscope may have a relatively small screen, which may impact a user experience of a clinician, such as viewing the screen of the video laryngoscope while also viewing a screen of the anesthesia device. There is not currently a provision for directly connecting the video laryngoscope to an electronic medical record system that includes additional information about the patient. The following description relates to an intubation device that integrates intubation administration tools, such as anesthesia and video laryngoscopy (VL) systems, and information from additional patient insight and data sources, in a single device. Insights from an anesthesia system, a VL system, additional imaging devices, and further patient insight capture and/or storage devices may be displayed at the same time, and optionally in real-time, on a display of the intubation device. Further, the intubation device may aggregate and process insights from integrated systems to generate additional insights and intubation control parameters. The intubation device enables an increased accuracy of intubation and a decreased time demand of intubation.

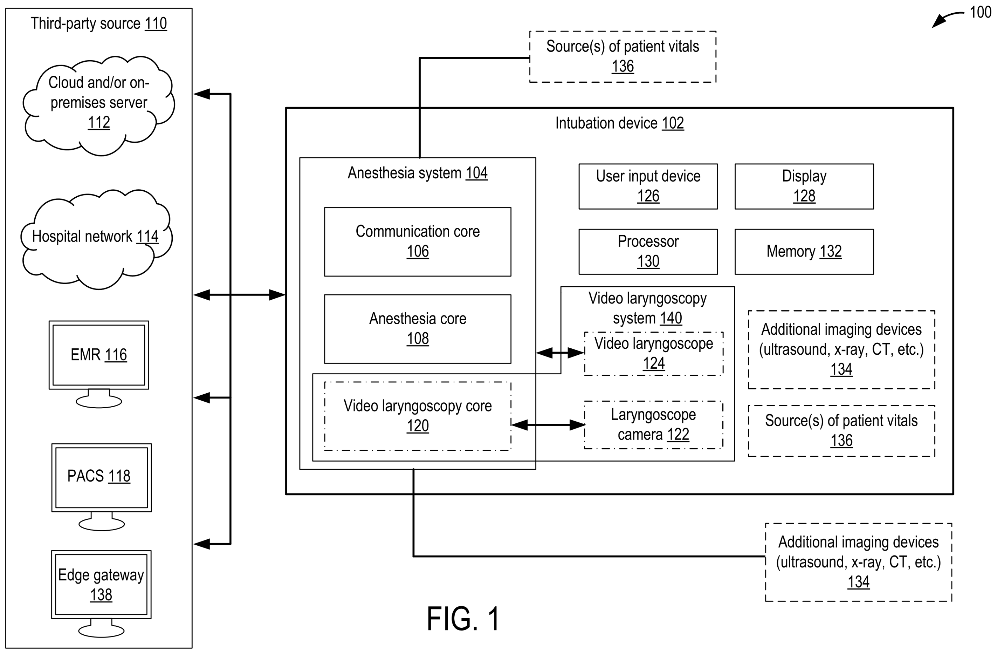

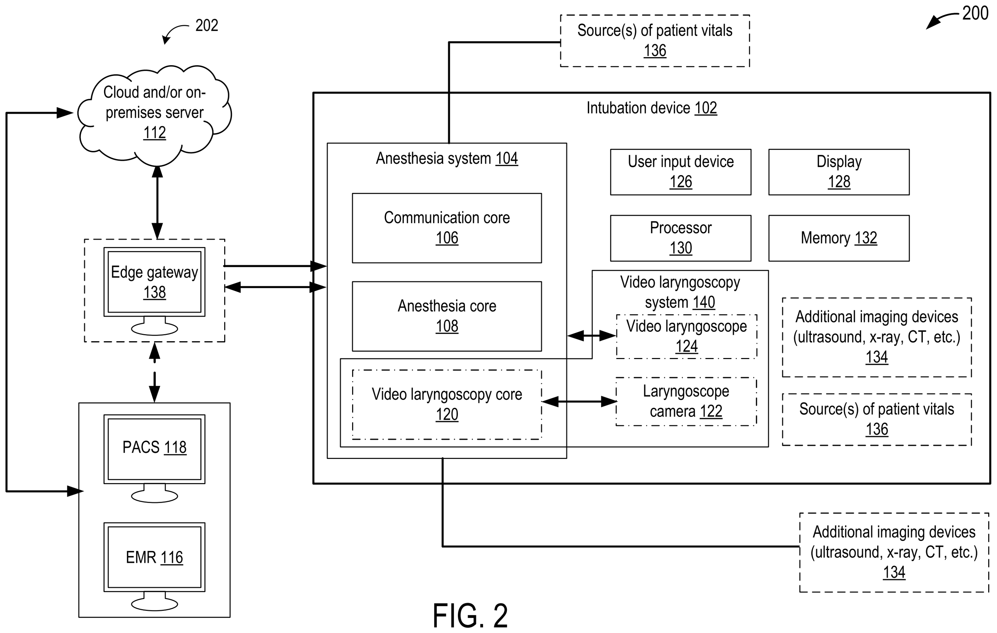

FIG. 1 shows a block diagram of an intubation system 100 including an intubation device 102 including two or more integrated intubation administration tools and additional patient insights and data sources, herein collectively referred to as "integrated systems". The intubation administration tools may be used to intubate and/or assist with intubation of a subject (herein, "subject" and "patient" may be used interchangeably). The intubation device 102 includes an anesthesia system 104 and a VL system 140 as examples of intubation administration tools of the integrated systems. The additional patient insights and data sources may store and/or generate information about the subject that may be related to, and thus assist with informing a process of, subject intubation. The intubation device 102 may include one or more of an additional imaging device 134, one or more of a source of patient vitals 136, and one or more of a third-party source(s) 110 as examples of additional patient insights and data sources. The intubation device 102 may have multiple possible configurations, where elements of the intubation device 102 are communicably coupled in different ways, as described herein. Further, the intubation device 102 may include additional and/or alternative integrated systems, as further described herein with respect to FIGS. 2-9. It is to be understood that the configurations of the intubation device 102 described herein are non-limiting, and other configurations may be possible without departing from the scope of the present disclosure. By integrating intubation administration tools (e.g., the anesthesia system 104 and the VL system 140) and additional patient insights and data sources (e.g., the additional imaging device(s) 134, the sources of patient vitals 136, and/or the third-party source(s) 110), the intubation device 102 is configured to provide guidance prior to and during intubation. Benefits of the systems and methods described herein include a simplified display output which may simplify a user experience, clinical insights that provide safer and faster intubation, and enable record keeping in EMR/PACS of anesthesia data, VL data, and other intubation insights, which may be used for evidence in future investigation, training, analysis. Further, by integrating the anesthesia system 104 and the VL system 140 into a single device, the intubation device provides a smaller footprint than two separate devices, and thus increased space management in an operating room.

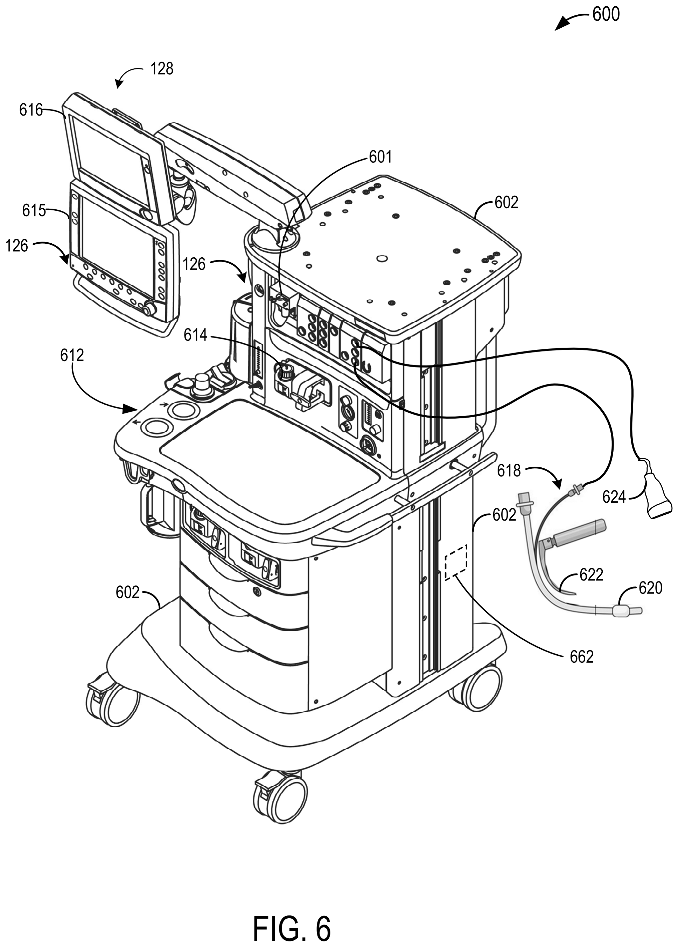

The anesthesia system 104 may be configured to deliver anesthesia to a subject, and to monitor the subject before, during, and after anesthesia administration. The anesthesia system 104 may include a communication core 106 and an anesthesia core 108. The communication core 106 may include both hardware and software that enable the intubation device 102 to send and receive data, such as analytics and insights, to and from one or more of the third-party source(s) 110, the source(s) of patient vitals 136, and/or the additional imaging device(s) 134. The third-party source(s) 110 may include one or more of a cloud server and/or on-premises server 112, a hospital network 114, an electronic magnetic records (EMR) server 116, a picture archiving and communication system (PACS) 118, and an edge gateway 138. The cloud server and/or on-premises server 112 may be configured to generate and/or store analytics and insights for intubation. The PACS 118 may additionally or alternatively include another type of picture and/or video archiving system. The anesthesia core 108 may include elements that are conventionally included in an anesthesia device, such as an electronic controller configured to control one or more operating parameters of the anesthesia device (e.g., control a type, rate, and/or amount of gas being provided to a subject). The anesthesia core 108 may include both hardware and software elements. Further detail describing anesthesia function of the intubation device 102 is described with respect to FIG. 6. The edge gateway 138 may have a function that connects internet of things (IoT) devices to edge infrastructure by translating IoT protocols into data formats that can be aggregated and processed at the edge. For example, the edge gateway 138 may enable communication between the anesthesia system 104 and the cloud server and/or on-premises server 112.

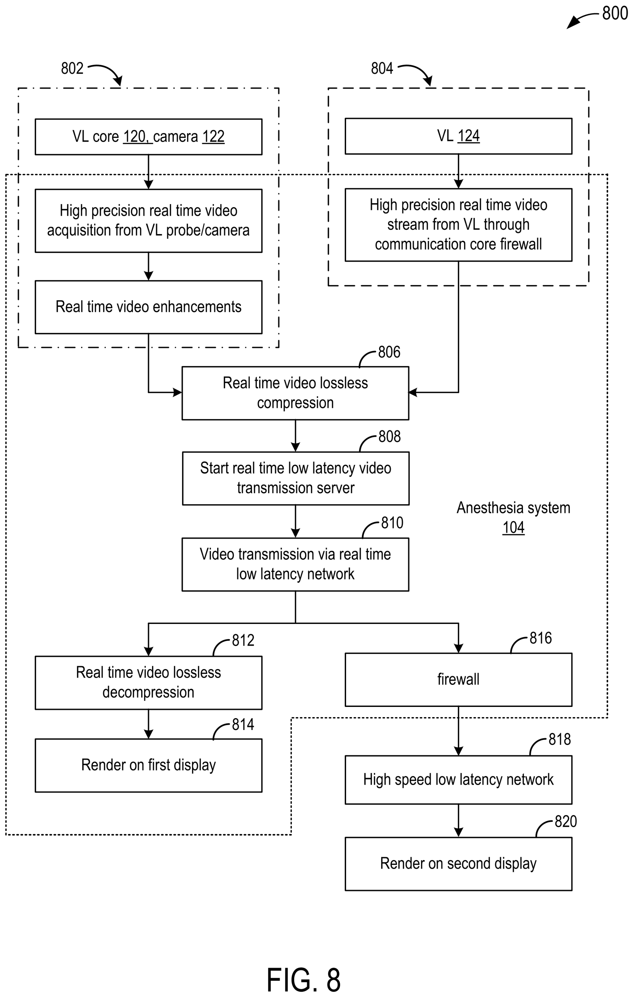

The VL system 140 may be configured to capture a laryngoscopy video of the subject and generate insights using the laryngoscopy video. The VL system 140 of the intubation device 102 may be integrated in the intubation device 102 in one or more configurations. For example, the VL system 140 may include a video laryngoscopy (VL) core 120 and a laryngoscope camera 122 that are integrated in the anesthesia system 104. The laryngoscope camera 122 may be a conventional device used to perform a video laryngoscopy. The laryngoscope camera 122 may be coupled to the VL core 120 via a wired and/or a wireless connection. The VL core 120 may include software that enables the intubation device 102 to perform functions of a conventional video laryngoscope. For example, the VL core 120 may use a laryngoscopy video captured by the laryngoscope camera 122 to generate insights about a subject airway, as further described with respect to FIGS. 8-18. The VL core 120 may also include software and hardware configured to process data received from the third-party source(s) 110 and generate insights and intubation guidance using data from the laryngoscope camera 122 and data from the third-party source(s) 110. For example, the VL core 120 may identify a desired intubation tube length and position of the intubation tube in the subject airway using the laryngoscopy video and imaging data (e.g., a CT image) captured by the additional imaging device(s) 134. In other configurations of the intubation device 102, the VL system 140 may be a video laryngoscope 124 that is communicably coupled to the anesthesia system 104 via a wired and/or a wireless connection. The video laryngoscope 124 may be an example of a conventional video laryngoscope, and may include conventional elements thereof, including a camera (e.g., a laryngoscope camera). The video laryngoscope 124 may be directly interfaced with the anesthesia system 104 via the communication core 106.

The additional imaging device(s) 134 may be an ultrasound device, an X-ray device, a computed tomography (CT) device, and so on. The additional imaging device(s) 134 may be directly interfaced with the anesthesia system 104 via the communication core 106. The source(s) of patient vitals 136 may include a patient monitor and/or the EMR 116, for example. In some configurations of the intubation device 102, one or more of the additional imaging device(s) 134 and/or one or more of the source of patient vitals 136 may be communicably coupled to the intubation device 102 via a wired and/or wireless connection (e.g., to the communication core 106).

The intubation device 102 may further include a user input device 126, a display 128, a processor 130, and a memory 132. The user input device 126 may include one or more analog and/or digital adjustment tool, such as knobs, switches, dials, keyboard, mouse, touch screen, and so on. The display 128 may be one or more screens or other visual display, such as a touch screen. The display 128 may, at the same time, output two or more of anesthesia data (e.g., from the anesthesia core 108), video laryngoscopy data (e.g., from the VL system 140), patient insights generated by the VL core 120 and/or received from the third-party source(s) 110, data captured by the additional imaging device(s) 134, and data retrieved from the PACS 118 and/or EMR 116, such that multiple data are shown on the display 128. For example, the display 128 may output a video laryngoscopy video stream (a VL stream), patient insights, vitals, and so on. The intubation device 102 thus provides synchronized visualization of anesthesia and VL data. Additional examples of the user input device 126 and display 128 are described with respect to FIGS. 6-7. The processor 130 may be operatively coupled to the memory 132, the communication core 106, the anesthesia core 108, and the VL system 140. The memory 132 may be a non-transitory computer-readable medium and may be configured to store computer executable code (e.g., instructions) to be processed by the processor 130 in order to execute one or more routines. The memory 132 may also be configured to store data received by the processor 130. For example, the memory 132 may store computer-readable instructions that may be executed by the processor 130 to generate intubation guidance from data generated and/or captured by the anesthesia system 104 and the VL system 140, and data received from the third-party source(s) 110. Further, the processor 130 may render data from the third-party source(s) 110, data generated and/or captured by the anesthesia system 104 and the VL system 140, and intubation guidance on the display 128 at the same time.

FIG. 2 shows a block diagram 200 of elements of the intubation system 100 and the intubation device 102 integrated in a first example communications network 202. When integrated in the first example communications network 202, the anesthesia system 104 of the intubation device 102 may act as a gateway for the intubation device 102 to communicate with one or more of the third-party source(s) 110 to access pre-op data. As a gateway, the anesthesia system 104 may be configured with hardware and/or software (e.g., the communication core 106) that facilitates data flow between different networks (e.g., the intubation device 102 and one or more of the third-party source(s) 110, the source(s) of patient vitals 136, and/or the additional imaging device(s) 134), thus acting as both a translator and connector for various protocols and data formats. The pre-op data may be stored in one or more of the EMR 116, the cloud server and/or on-premises server 112, and the PACS 118. Pre-op data may include data from a pre-op procedure (e.g., from one or more of an x-ray, a blood test, and so on), patient history, prescriptions, special patient conditions, clinician information, and so on. The cloud/on-premises server 112 may provide additional insights that are generated (e.g., by a computing device communicably coupled to the cloud/on-premises server 112) from the pre-op data, such as optimal intubation tube length from a clinician chosen reference point.

In the example of FIG. 2, the anesthesia system 104 may directly and/or indirectly access pre-op data and additional insights via the cloud server and/or on-premises server 112. The communication core 106 may be a bridge that connects the intubation device 102 to the cloud server and/or on-premises server 112. The communication core 106 may further serve as a single connection between the intubation device 102 and the EMR 116 and/or PACS 118. The edge gateway 138 may be optionally included and act as an intermediate communicator between the anesthesia system 104 of the intubation device 102 and the cloud server and/or on-premises server 112. When the edge gateway 138 is included, pre-op data may be accessed by the anesthesia system 104 via the edge gateway 138. When the edge gateway 138 is not included, pre-op data may be accessed by the anesthesia system 104 via the cloud/on-premises server 112. Insights may be generated, collated, and present in the cloud server and/or on-premises server 112. For example, the anesthesia system 104 may be in communication with the cloud server and/or on-premises server 112, which may further be in communication with respective devices like the PACS 118 via a single path communication, which may reduce complexity of data transmission to and from the intubation device 102.

Via the communication core 106, the anesthesia system 104 may act as both a gateway and a consumer of data from third-party sources 110. For example, the communication core 106 of the anesthesia system 104 may send and receive analytics and insights from the cloud server and/or on-premises server 112. The anesthesia system 104 may send the data from one or more additional imaging devices(s) 134 to the PACS 118 or retrieve the data in the PACS 118 that is generated as part of the pre-op checkup (e.g., CT images). The same data can also be made available in the cloud server and/or on-premises server 112 or retrieved therefrom. The same is applicable for EMR data from the EMR 116. The intubation device 102 may synchronize output pre-op data and additional insights received from the cloud server and/or on-premises server 112, and anesthesia data (e.g., anesthesia vitals/monitoring parameters), data from additional imaging devices (e.g., imaging data), and VL data (e.g., a VL stream).

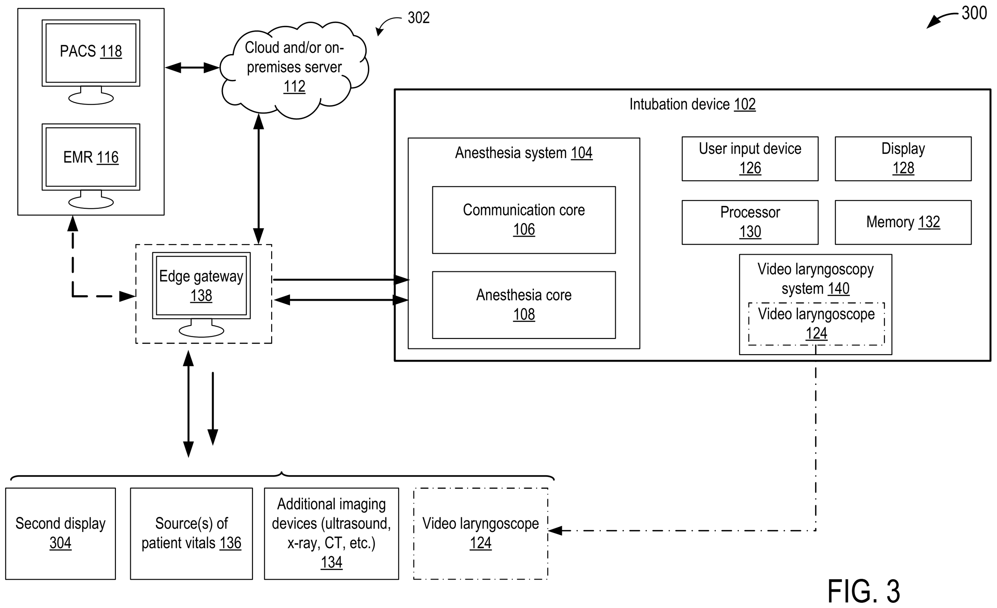

FIG. 3 shows a block diagram 300 of elements of the intubation system 100 and the intubation device 102 integrated in a second example communications network 302. Similar to the first example communications network 202, the edge gateway 138 may be optionally included. The intubation device 102 may be in direct communication with the cloud server and/or on-premises server 112, or in indirect communication with the cloud server and/or on-premises server 112 via the edge gateway 138. In the second example communications network 302, the source(s) of patient vitals 136, additional imaging device(s) 134, and a second display 304 are communicably coupled to the intubation device 102 via the cloud server and/or on-premises server 112, and optionally via the edge gateway 138, rather than through direct (e.g., wired and/or wireless) communication. The second display 616 may be a mobile or stationary display, such as a computer monitor, handheld mobile device, television screen, and so on. The video laryngoscope 124 may also be communicably coupled to the anesthesia system 104 via the cloud server and/or on-premises server 112, and optionally via the edge gateway 138. Additional or alternative devices that are communicably coupled to the hospital network 114 via the cloud server and/or on-premises server 112, and/or the edge gateway 138, may also be coupled to the intubation device 102 in this way. Real-time protocols over network may be used to achieve real-time performance with low latency on high-speed low latency networks. Third-party source(s) 110 that are communicably coupled to the intubation device 102 via the communication core 106 may be authenticated by the anesthesia system 104. The cloud server and/or on-premises server 112, and/or the edge gateway 138 may act as an integrator for the third-party source(s) 110 and/or additional devices attempting to communicably connect to the intubation device 102.

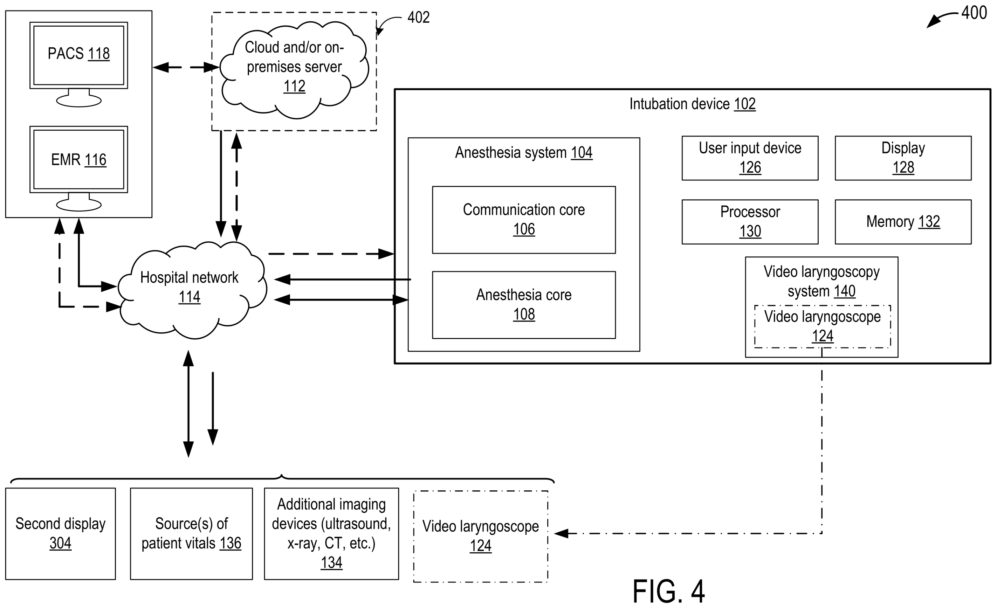

FIG. 4 shows a block diagram 400 of elements of the intubation system 100 and the intubation device 102 integrated in a third example communications network 402. The third example communications network 402 is similar to the second example communications network 302, however the cloud server and/or on-premises server 112, and/or the edge gateway 138 may not be used as an integrator. Instead, the third-party source(s) 110 and/or additional devices such as the source(s) of patient vitals 136, additional imaging device(s) 134, and a second display 304 may be communicably coupled to the intubation device 102 via the hospital network 114, rather than through direct (e.g., wired and/or wireless) communication. The video laryngoscope 124 may also be communicably coupled to the anesthesia system 104 via the hospital network 114 (e.g., via the cloud server and/or on-premises server 112 and/or via the edge gateway 138). The hospital network 114 may be a high-speed, low latency network. Various permutations and combinations of certain third-party sources 110 may demand a minimum of one device on the hospital network 114 and certain devices directly interfaced to the intubation device 102, and vice versa. Real-time protocols over the hospital network 114 may be used to achieve real-time performance with low latency on high-speed low latency networks. In implementations where the edge gateway 138 and the cloud server and/or on-premises server 112 are excluded, anesthesia system 104 may operate as a data aggregator. With newer standard protocols like HL7-IHE, the anesthesia system 104 may directly communicate with various devices (e.g., the EMR 116 and/or the PACS 118) to retrieve data like pre-op checkup data, patient history data, and so on. In conventional examples, this data was accessed through a centralized repository such as a cloud/on-premises server where the back end performed data collection and generation of analytics and insights. In this implementation, this is performed by the anesthesia system 104 which functions as an aggregator and source for analytics and insights, which are generated by the anesthesia system 104. The devices in this example can be connected through the hospital network 114 or directly to the anesthesia system 104. All permutations and combinations of this concept may be possible. When the anesthesia system 104 directly communicates with devices like PACS 118 to receive the insights and update/add additional information to generate more meaning full insights to be consumed by the user during intubation, the cloud/on-premises server 112 may not be used.

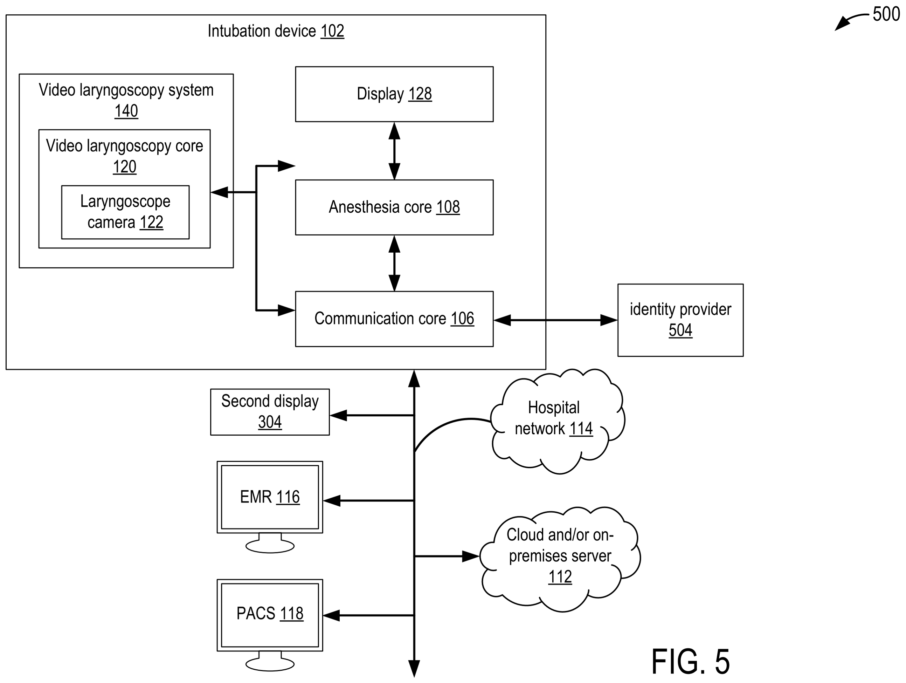

FIG. 5 shows a block diagram of elements of the intubation system 100 and the intubation device 102 integrated in a connectivity system architecture 500. For record keeping and security, authentication and record keeping may use data for trainings, investigations, and so on. The block diagram highlights the different components involved in integration of VL video with other clinical parameters and alarms. This is one example implementations, but is not limited to the architecture of FIG. 5. Video transmission may use real time low latency network to send the VL video stream to the display 128. Clinical parameters and alarms may be streamed using standard or proprietary protocols. To achieve higher security, the VL video stream may be sent to the second display 304 after authentication. Consumers of the integrated data may include and are not limited to the second display 304, EMR 116, PACS 118, cloud server and/or on-premises server 112, which may further be used to store images and/or data for clinical evidence, clinical training purposes, and so on. The communication core 106 may communicate with an identity provider 504 that provides information about authenticity of devices attempting to communicably connect with the intubation device 102.

FIG. 6 shows a perspective view of an example of an intubation device 600, which is a non-limiting example of the intubation device 102 of FIGS. 1-5. The intubation device 600 has an external housing 602 that may at least partially enclose elements of the intubation device 102 described with respect to FIG. 1. For example, the processor 130 and the memory 132 may be positioned internally relative to the external housing 602 of the intubation device 600. The display 128 and/or the user input device 126 may be integrated as part of the external housing 602, such that a user may interact with the display 128 and the user input device 126. Further, at least part of the anesthesia system 104 and the VL system 140 may be positioned internally, relative to the external housing 602.

The intubation device 600 includes elements of an anesthesia system (e.g., the anesthesia system 104), including a respiratory gas module 601, a ventilator 612, and a vaporizer 614. When a patient breathing circuit is coupled to the ventilator 612, the breathing gases (e.g., fresh gas, oxygen, and/or nitrous oxide mixed with vaporized anesthetic agent) exit the intubation device 600 from an inspiratory port and travel to a patient. Expiratory gases from the patient reenter the intubation device 600 via an expiratory port, where the carbon dioxide may be removed from the expiratory gases via the absorber canister. The vaporizer 614 includes a housing having a drug reservoir that contains a supply of anesthetic agent to be delivered to a patient. The drug reservoir may have a discharge opening formed in a back wall of the housing that is configured to receive a discharge tube (not shown), which is part of the anesthesia machine, forming a gas-tight seal for delivery of anesthetic vapor to a patient. During operation of the vaporizer 614, an operator (e.g., an anesthesiologist) may adjust an amount of vaporized anesthetic agent that is supplied to a patient by adjusting a flow rate of gases from the gas source(s) (e.g., the gas pipelines) to the vaporizer. The flow rate of the gases from the gas source to the vaporizer may be adjusted by the operator via adjustment of one or more flow adjustment devices. For example, the flow adjustment devices may include analog and/or digital adjustment dials and/or other user input devices configured to actuate one or more flow control valves of the intubation device 600. In one example, a first flow control valve may be positioned between the gas source(s) and the vaporizer 614 and may be actuatable via the flow adjustment devices to a fully opened position, a fully closed position, and a plurality of positions between the fully opened position and the fully closed position.

The intubation device 600 further includes elements of a VL system 618 (e.g., the VL system 140 of FIGS. 1-5). In the example of FIG. 6, the VL system 618 includes a laryngoscope 620 and a blade 622 that are configured to be inserted into an oral cavity (e.g., mouth) of a subject to hold down the tongue of the subject while also lifting up the epiglottis of the subject. The laryngoscope 620 may be selectively and/or fixedly coupled to the blade 622. The blade 622 and/or the laryngoscope 620 may include a high-resolution, waterproof camera. The blade 622 and the laryngoscope 620 may include an electronics package that is configured to couple the camera to the intubation device 600 via a wired and/or a wireless connection. When the blade 622 and the laryngoscope 620 are inserted into the oral cavity of the subject, the camera may capture images and/or a real-time video stream of the airway of the subject. "Real-time" as used herein describes that a first operation (e.g., capturing data) occurs without intentional delay and at as close to the same time as is possible as occurrence of a second operation (e.g., output of the captured data). As further described herein, the real-time video stream may be rendered with anesthesia data (captured by the anesthesia system 104) at the same time on a display of the intubation device 600.

In some configurations, as described with respect to FIGS. 1-5, the intubation device 600 may include one or more of an additional imaging device. In the example of FIG. 6, elements of an ultrasound imaging device, such as an ultrasound probe 624, are included in the intubation device 600. The ultrasound probe 624 may be coupled to the intubation device 600 via a wired and/or a wireless connection. Images captured by the ultrasound probe 624 may be rendered in real-time alongside data captured by the anesthesia system 104 and the VL system 618 (e.g., the camera of the blade 622 and the laryngoscope 620) on the display of the intubation device 600.

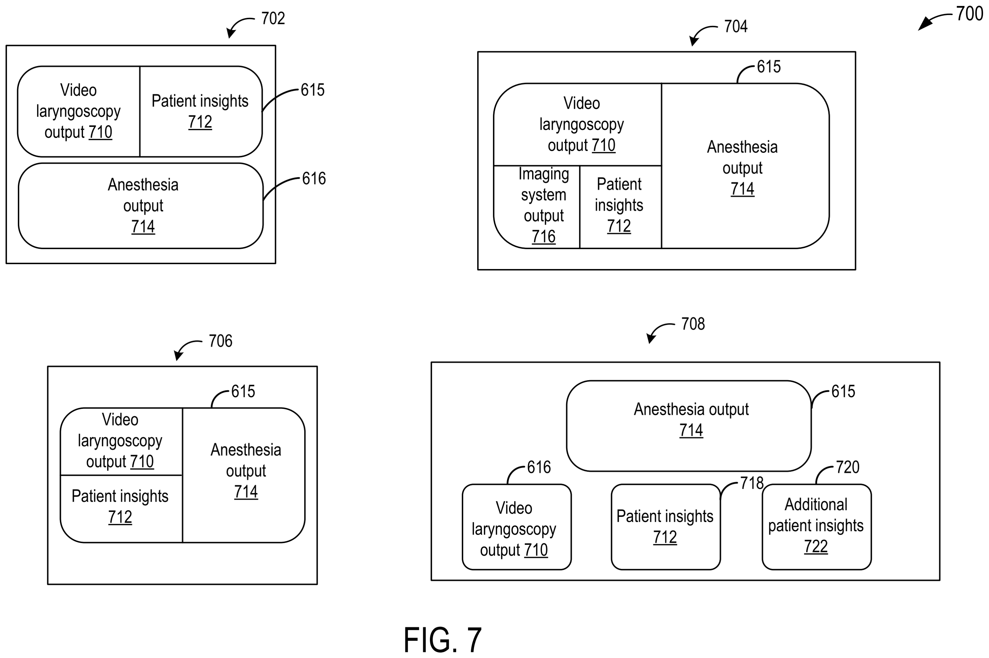

The display of the intubation device 600 may include a first display device 615 and a second display device 616. Each of the first display device 615 and the second display device 616 may be examples of the display 128 of FIGS. 1-5. Synchronized visualization of anesthesia data and video laryngoscopy data may be provided via the first display device 615 and/or the second display device 616, as further described herein with respect to FIGS. 7-18.

In some examples, one or more operating parameters of the intubation device 600 may be controlled via an electronic controller 662 of the intubation device 600. The controller 662 is shown as a dashed-line box in FIG. 6, indicating that the controller 662 and elements thereof are positioned internally relative to the external housing 602. The controller 662 may include the processor 130 operatively connected to the memory 132, as described with respect to FIGS. 1-5. The memory 132 may be a non-transitory computer-readable medium and may be configured to store computer executable code (e.g., instructions) to be processed by the processor 130 in order to execute one or more routines. The memory 132 may also be configured to store data received by the processor 130. The controller 662 may be communicatively (e.g., wired or wirelessly) coupled to one or more external or remote computing devices, such as one or more of the third-party source(s) 110, and may be configured to send and receive various information, such as measured patient monitoring parameters, electronic medical record (EMR) information, procedure information, and so forth.

The controller may receive signals from the various sensors of the intubation device 600 and employs the various actuators of the intubation device 600 to adjust operation of the intubation device 600 based on the received signals and instructions stored on the memory 132 of the controller 662. For example, the flow of gases to the inspiratory port may be controlled via an input device (e.g., keyboard, touchscreen, etc.) coupled to the electronic controller of the intubation device 600. The controller 662 may be electrically coupled to display device 615 and/or 616 in order to display operating parameters of the intubation device 600. The controller may receive signals (e.g., electrical signals) via the input device and may adjust operating parameters of the intubation device 600 in response (e.g., responsive) to the received signals. For example, the operator may input a desired flow rate of gases (e.g., oxygen) flowing from the gas source to the patient and/or vaporizer 614. Controller 662 is shown in FIG. 6 for illustrative purposes, and it is to be understood that controller 662 may be located internally of intubation device 600 and thus may not be visible externally on anesthesia machine. Controller 662 may include multiple devices/modules that may be distributed across intubation device 600.

FIG. 7 shows example display configurations 700 of the intubation device 102, 600. As briefly described with respect to FIG. 6, the display 128 of the intubation device 102, 600 may include one or more of a display screen, such as the first display device 615 and the second display 616. The intubation device 102, 600 is configured to render data from one or more of the third-party source(s) 110, data generated and/or captured by the anesthesia system 104 and the VL system 140 (e.g., the VL core 120 and the laryngoscope camera 122, and/or the video laryngoscope 124), and intubation guidance at the same time on the display 128. It is to be understood that "alongside" as used herein means at the same time as, such that multiple types of data are displayed at the same time to provide simultaneous visualization thereof. A first example display configuration 702 includes a VL output 710 (e.g., a real-time laryngoscopy video) and patient insights 712 (e.g., pre-op checkup data, patient history, and so on) on the first display device 615. The patient insights 712 may be received by the intubation device 102, 600 from one or more of the third-party sources 110, such as the EMR 116, the PACS 118, and so on. The first example display configuration 702 further includes an anesthesia output 714 (e.g., a rate, type, and/or amount of anesthesia gas provided to the subject) on the second display 616. A second example display configuration 704 includes the VL output 710, patient insights 712, the anesthesia output 714, and an imaging system output 716 on the first display device 615. The imaging system output 716 may be received by the intubation device 102, 600 from one or more of the additional imaging device(s) 134, and may include one or more of a real-time and/or recorded ultrasound image, X-ray image, CT image, and so on. A third example display configuration 706 includes the VL output 710, patient insights 712, and the anesthesia output 714 on the first display device 615. A fourth example display configuration 708 includes the anesthesia output 714 on the first display device 615, the VL output 710 on the second display 616, patient insights 712 on a third display 718, and additional patient insights 722 on a fourth display 720. The additional patient insights 722 may be retrieved by the intubation device 102, 600 from the same or different third-party source(s) 110 as the patient insights 712. One or more of the second display 616, the third display 718, and the fourth display 720 may be a mobile or stationary display, such as a computer monitor, handheld mobile device, television screen, and so on. The example display configurations 700 shown in FIG. 7 are non-limiting, illustrative examples of how multiple types of data from one or more sources included in and/or communicably coupled to the intubation device 102, 600 may be displayed at the same time on one or more displays of the intubation device 102, 600.

FIG. 8 shows an example representation of a system architecture 800 of the intubation device 102, 600. FIG. 8 illustrates how different components may be involved in processing the VL video stream to prepare and output rendering of the VL video stream on the display 128 of the intubation device 102, 600. Instructions for executing operations of the system architecture 800 may be stored in the memory 132 and executed by the processor 130 of the intubation device 102, 600, unless otherwise noted. Two example architectures are shown, which correspond to configurations of the VL system 140 in the intubation device 102, 600. When the VL system 140 is configured as a VL core 120 with the laryngoscope camera 122, a first architecture 802 is implemented. The first architecture 802 includes acquiring high precision, real-time video from the laryngoscope camera 122. The processor 130 may perform real-time video enhancements, such as color correction, applying contrast, and so on. The second architecture 804 may be implemented when the VL system 140 is configured as the video laryngoscope 124 communicably coupled to the anesthesia system 104 via the communication core 106. VL video stream acquisition may not be demanded since the video laryngoscope 124 performs acquisition methods (e.g., a VL core is present in the video laryngoscope 124, rather than in the anesthesia system 104). The second architecture 804 includes streaming the VL video stream captured by the video laryngoscope 124 to the processor 130 via a firewall of the communication core 106. Thus, both the first architecture 802 and the second architecture 804 enable mass storage data sharing (e.g., of video files) between the VL system 140 and the anesthesia system 104.

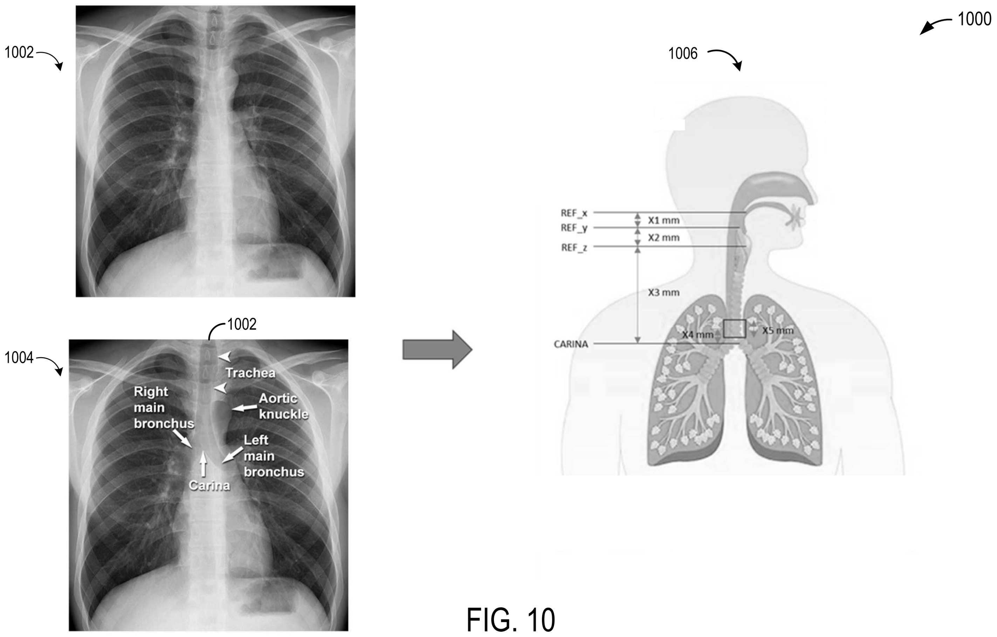

Additionally, the VL core 120 may be used to set and monitor parameters in a VL process (e.g., inserting the laryngoscope camera 122 into the airway of the subject). The anesthesia system 104 may also communicate with the VL core 120 (e.g., two-way communication between the VL core 120 and the anesthesia system 104) to send data in real-time from the anesthesia system 104 to the VL core 120. For example, the anesthesia system 104 may act as a gateway for communication with the third-party source(s) 110, the additional imaging device(s) 134 and/or the source(s) of patient vitals 136. The anesthesia system 104 may retrieve and/or receive data from the additional patient insights and data sources and send retrieved data as well as anesthesia data from the anesthesia system 104 to the VL core 120. For example, the data sent from the anesthesia system 104 to the VL core 120 may include patient vital signs, anesthesia oxygen percentage settings, time elapsed since a start of an intubation procedure, and/or information elaborated from the intubation device, such as a distance from an intubation tube to a carina. The VL core 120 may process this data along with data captured by the VL system 140 (e.g., laryngoscopy video feed, recorded and/or real-time) to generate further patient insights, as will be described with respect to FIGS. 10-18.

Both the first architecture 802 and the second architecture 804 may implement the following steps of the system architecture 800. To efficiently render the VL video stream via low latency network, the VL video stream undergoes video lossless compression and decompression techniques. The system architecture 800 applies a real-time video lossless compression at 806. At 808, the system architecture 800 starts a real-time, low latency video transmission server. This may include opening a communication channel, via the communication core 106, with the display 128. At 810, the system architecture 800 begins video transmission of the VL video stream in real-time via the low latency network. The system architecture 800 may output at the same time (e.g., in real time) the VL video stream to more than one displays of the intubation device 102, 600. For example, at 812, the system architecture 800 executes real-time video lossless decompression on the VL video stream, and at 814, renders the VL video stream on the first display (e.g., the first display device 615). The system architecture 800 also outputs the VL video stream to the firewall 816 (e.g., of the communication core 106). To achieve higher security, the VL video stream is sent to the second display 304 via a high secured firewall module. Via the high speed low latency network 818, the VL video stream may be transmitted to and rendered on the second display 820 (e.g., the second display device 616 and/or the second display 304).

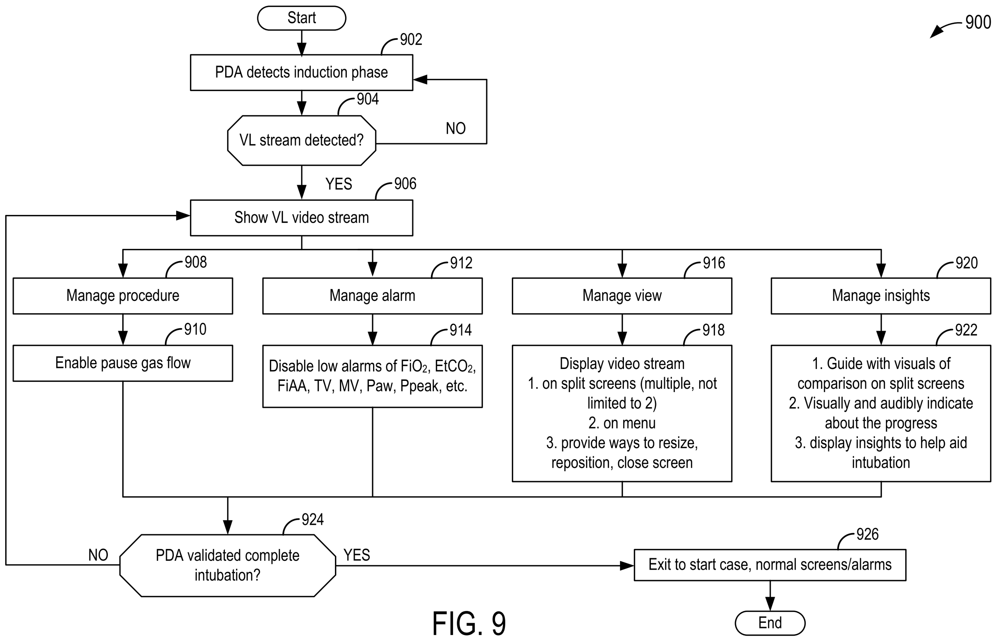

FIG. 9 shows a flow chart for a method 900 for intubation control parameters and anesthesia phase detection during intubation. The method 900 may be executed by the intubation device 102, 600 of FIGS. 1-5 and 6, respectively. The method 900 integrates display of anesthesia data and VL video stream on the display, including resizing of display elements and integration of pre-op insights on the display. At 902, the method 900 includes detecting an induction phase using a phase detection algorithm (PDA). The PDA may be a conventional PDA that is stored in the memory of the intubation device. At 904, the method 900 includes determining if a VL video stream (e.g., captured by the VL system 140 of the intubation device 102, 600) is detected. The VL video stream may be acquired as described with respect to FIG. 8. If the VL video stream is not detected, the method 900 returns to 902 to continue executing the PDA for intubation phase detection. If the VL video stream is detected, the method 900 proceeds to 906 to show the VL video stream on the display of the intubation device. Anesthesia data may be rendered on the display at the same time as the VL video stream (e.g., in real-time). Following 906, one or more operations may be executed to control operation of the intubation device based on the anesthesia data and VL video stream. At 908, the method 900 may include operations to manage intubation and/or anesthesia procedures. For example, at 910, the method 900 may enable and/or disable pause of gas flow. For example, if anesthesia gas flow to the subject is active, the method 900 may enable the gas flow to be passed and disable alternative oxygen gas supply. At 912, the method 900 may include operations to manage alarms of the intubation device. This may provide ways to enable/disable induction phase alarm settings. For example, at 914, the method 900 may include disabling FiO2 low, EtCo2, 20 sec APNEA, TV/MV low, agent low, and airway pressure low alarms. In other examples, the method 900 may include enabling pause gas flow count down, keep 60 seconds APNEA, and outputting numeric indicators of FiO2, EtCo2, gas flow, and/or airway pressure. At 916, the method 900 may include operations for managing a view shown on the display. For example, at 918, the method 900 may include enabling selection among screen views, such as those shown in FIG. 7. The video stream may be displayed: on split screens (two or more), on a menu, or the view may provide ways to resize and/or reposition screen views, or to close the screen. The method 900 may output visual and/or audible alarm messages about the VL video and anesthesia data feed. At 920, the method may include operations for managing patient insights. This may assist in guiding a user with the visuals of comparison of the real time images against the reference images taken prior (e.g., pre-op data). For example, at 922, the method 900 may include: guiding a user to view various insights by outputting visuals of comparison on split screens; visually and/or audibly indicating whether the progress of the procedure is as per the reference or deviating; and/or displaying insights that may aid intubation. At 924, the method 900 includes determining if PDA has validated complete intubation (e.g., intubation tube positioned at desired location in airway). If complete intubation is not validated, the method 900 may return to 906 to continue showing the VL video stream. If complete intubation is validated, the method 900 proceeds to 926 to exit the display to start case, and show normal screens/alarms (e.g., show an operator-configured base screen display). The method 900 ends.

FIG. 10 shows examples 1000 of a chest X-ray, a labeled chest X-ray, and patient insights generated from the labeled chest X-ray. A chest x-ray 1002 is shown as an example of data that is captured by the additional imaging device(s) 134 and/or data that is stored in one or more third-party source(s) 110, such as the EMR 116 and/or the PACS 118. For example, the chest x-ray 1002 may be captured as part of a pre-op checkup. The chest x-ray 1002may be used to generate patient insights. As an example, intubation procedures may include placing an intubation tube in the trachea of the subject at a certain distance from the carina. The tube length used to position an end of the intubation tube at a desired location (e.g., the certain distance from the carina) may depend on anatomy of the patient, as well as a type of intubation procedure being performed. For some patients, the intubation tube may be extended into a branch of the trachea to ventilate a single lung. Additionally, patient position may influence a desired tube length used to achieve the desired position of the end of the intubation tube. A labeled chest x-ray 1004 includes indicators superimposed on the chest x-ray 1002 to indicate anatomies of interest. As examples, the labeled chest x-ray 1004 includes labels for the right main bronchus, the trachea, the aortic knuckle, the left main bronchus, and the carina. Conventionally, a healthcare provider may select a reference point on the chest x-ray 1002 and/or labeled chest x-ray 1004 that is used to determine a position at which to place an end of the intubation tube. The reference point may be, for example, the larynx, the epiglottis, and so on.

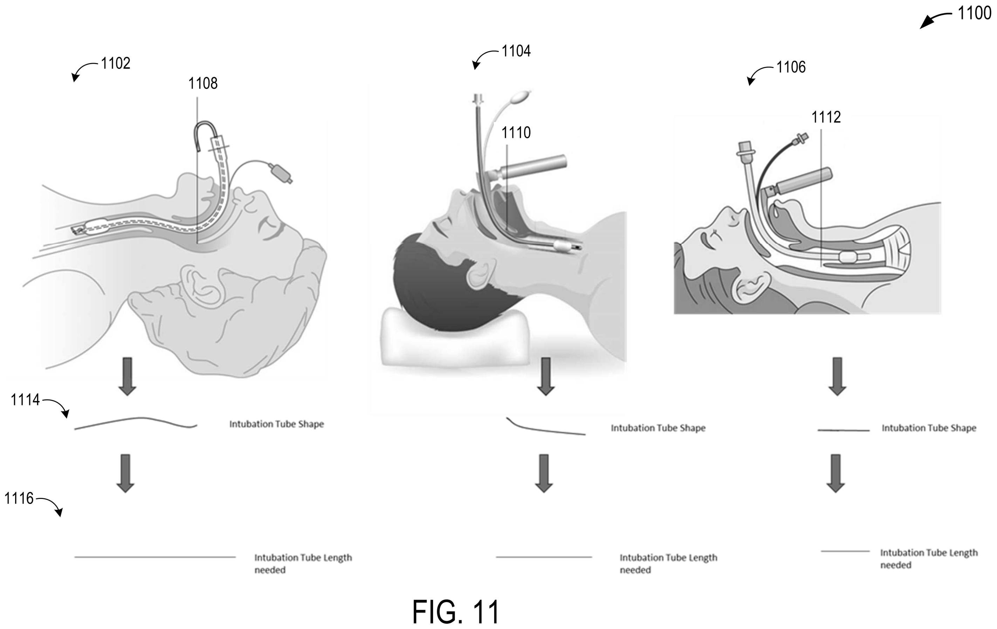

A reference diagram 1006 shows how the labeled chest x-ray 1004 may be used to determine a length of the intubation tube, with respect to the reference point. In the reference diagram 1006, "REF" refers to various reference points with respect to anatomy, where each reference point is based on a different tube length. For example, REF_x, REF_y, and REF_z each indicate a different reference point along the airway of the subject. The carina is also identified in the reference diagram 1006. Example distance multipliers are provided to assist in determining length of the intubation tube. For example, if REF_y is selected as the reference point, the intubation tube length may be calculated using the first equation (1): $Y \left(mm\right) = X 2 + X 3 - X 4$ (1). The reference diagram 1006 illustrates the distance to the carina as a linear dimension, though it is to be understood that the linear dimension includes factors based on curvature for a non-straight trachea, as further described with respect to FIG. 11.

FIG. 11 shows illustrations 1100 of a first intubation tube identifier 1102, a second intubation tube identifier 1104, and a third intubation tube identifier 1106. In the first intubation tube identifier 1102, a first reference point 1108 is used to identify a desired distance from the carina. The second intubation tube identifier 1104 uses a second reference point 1110, and the third intubation tube identifier 1106 uses a third reference point 1112. As is illustrated in FIG. 11, patient airways may have different shapes 1114, which result in different demanded intubation tube lengths 1116.

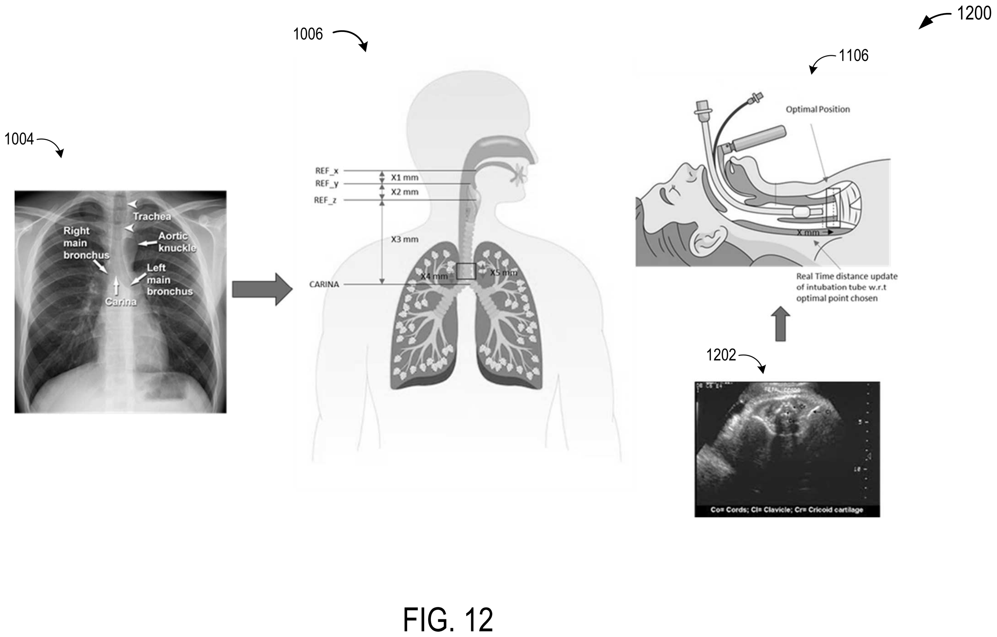

Insights provided by the reference diagram 1006 along with the VL video stream may help a clinician to have a predetermined intubation tube length with respect to the reference point inserted faster than having to identify a desired length for optimal placement during intubation, saving valuable time and making a faster and safer intubation. FIG. 12 shows an implementation 1200 where, along with the insights provided in FIGS. 10-11, an additional imaging device may be used during intubation that can give real time feed of the intubation tube with respect to a desired position of the intubation tube end point, along with real-time insights on distance the intubation tube must travel to reach the desired position. In the example of FIG. 12, the additional imaging device is an ultrasound that outputs an ultrasound image 1202, which is used to track the position of the intubation tube in real time with respect to the desired position. Other imaging devices may be used without departing from the scope of the present disclosure. These insights may decrease a time and complexity of the intubation process, which may increase a speed and accuracy of intubation by providing guidance during an intubation phase. In some examples, one or more of the chest x-ray 1002, the labeled chest x-ray 1004, and the reference diagram 1006 may be displayed alongside the VL video stream and the anesthesia data. The clinician may also access all these images (e.g., X-ray, other data used to generate insights) as requested. The system and method are described herein with respect to X-ray, however they may be extended to other imaging techniques such as ultrasound, CT, MRI, and so on.

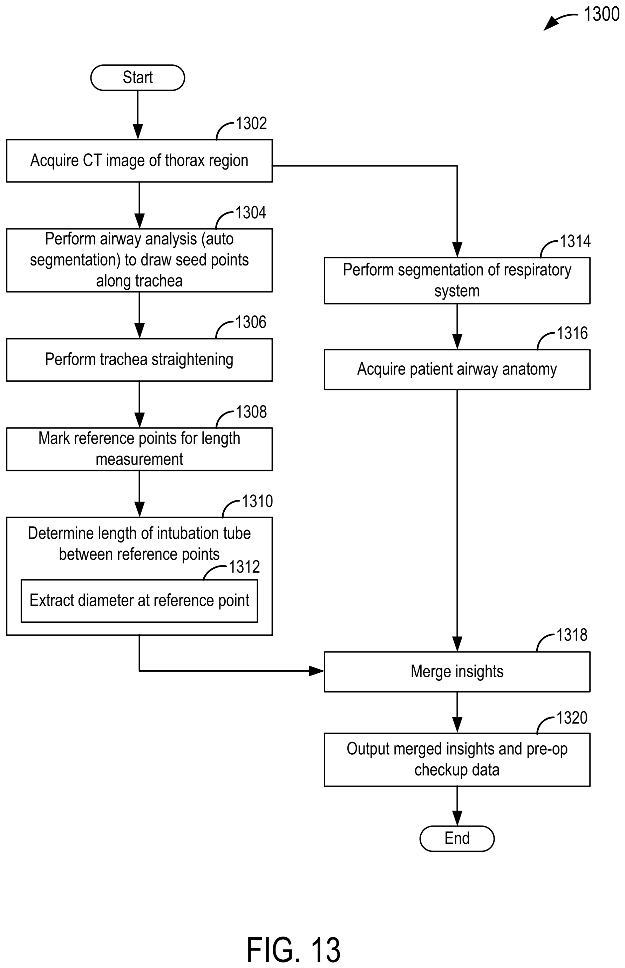

FIG. 13 shows a flow chart for a method 1300 for generating insights used to determine a tube length of an intubation tube. Instructions for executing the method 1300 may be stored in the memory of an integrated intubation device, such as the memory 132 of the intubation device 102, for example. The instructions may be executed by the processor 130 and/or the VL core 120 of the VL system 140.

At 1302, the method 1300 includes acquiring a CT image of a thorax region of a subject (e.g., a patient). The CT image may be captured by an imaging device (e.g., a CT system) that is communicably coupled to the intubation device 102. The imaging device may be an example of the additional imaging device(s) 134 that is integrated in and/or coupled to the intubation device 102. As described above, the anesthesia system 104, and specifically the communication core 106, may act as a gateway for the intubation device 102 and enable the intubation device 102 to request and receive data, including CT images.

At 1304, the method 1300 includes performing an airway analysis to draw seed points along a trachea of the thorax region in the CT image. The airway analysis may be an auto-segmentation procedure that uses artificial intelligence (AI) and/or machine learning (ML) to identify anatomy landmarks in the CT image, and label the CT image with reference points at regions of interest in the anatomy (e.g., a top of the trachea, a bottom of the trachea, and so on).

At 1306, the method 1300 includes performing trachea straightening. As described with respect to FIG. 11, the trachea may have a non-linear (e.g., curved) shape due to anatomy of the subject and/or positioning of the subject. To accurately identify a length of the trachea, it may be desirable to convert a non-linear shape of the trachea to a linear shape via trachea straightening, and measure the length of the trachea as the linear shape.

At 1308, the method 1300 includes marking reference points for length measurement of the trachea. As described with respect to FIGS. 10 and 12, the reference points may indicate a desired position of an end of the intubation tube with respect to anatomy landmarks, such as the carina.

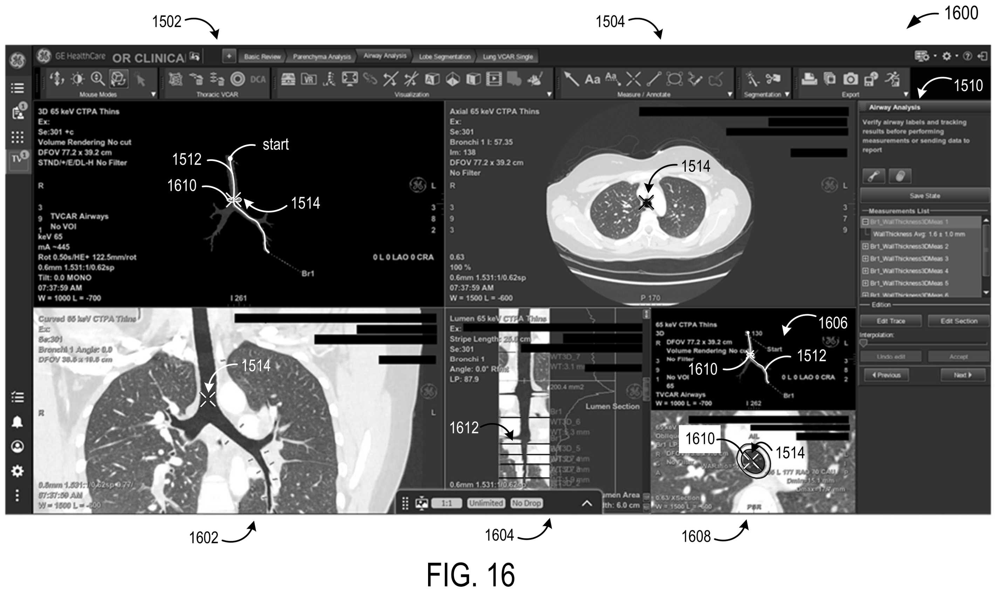

At 1310, the method 1300 includes determining the length of the intubation tube between reference points. The length of the intubation tube may be determined as described with respect to FIGS. 10-12. The method 1300 may further include extracting a diameter of the airway of the patient, and/or a desired diameter of the intubation tube, at one or more of the reference points, as further described with respect to FIG. 16.

Following acquisition of the CT image of the thorax region at 1302, at 1314, the method 1300 may include performing segmentation of the respiratory system of the subject. Operation 1314 and operation 1304 may be performed at the same time, or one after the other. A conventional segmentation procedure may be automatically executed to segment the CT image.

At 1316, the method 1300 includes acquiring the patient airway anatomy. A segmented CT image (e.g., from operation 1314) may be processed and compared to known anatomy landmarks (e.g., using AI and/or ML methods) to identify the patient airway anatomy.

At 1318, the method 1300 includes merging the insights acquired from the CT image of the thorax region. For example, the patient airway anatomy acquired at operation 1316, the length of the intubation tube determined at operation 1310, and the diameter of the intubation tube and/or the airway at one or more reference points extracted at operation 1312 may be combined into a single dataset/transferrable file.

At 1320, the method 1300 include outputting the merged insights and pre-op checkup data. The pre-op checkup data may be retrieved from the third-party source(s) 110 and/or the source(s) of patient vitals 136 by the anesthesia system 104. The pre-op checkup data and the merged insights may be output to the display 128 of the intubation device 102 and/or to the second display 304 communicably coupled to the intubation device 102 in real-time and at the same time (e.g., as illustrated in FIG. 7). In this way, intubation insights may be generated by the intubation device using data acquired by the intubation device from multiple sources, including intubation administration tools and additional patient insight and data sources. The intubation insights are made available (e.g., output for display and/or storage) during the intubation process.

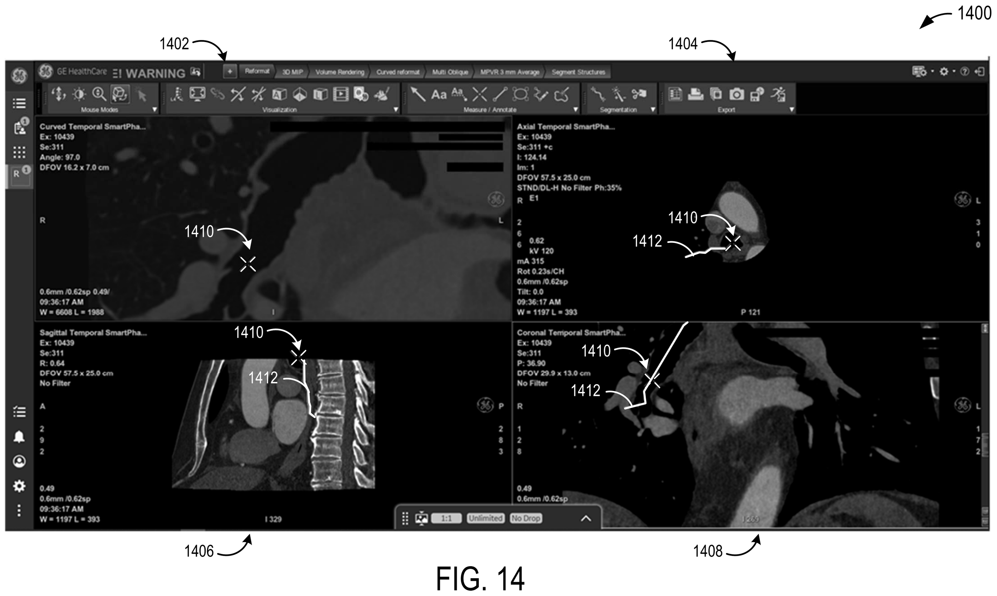

FIG. 14 shows CT images 1400 illustrating various views of an anatomical region of interest of a patient. The CT images 1400 include a curved temporal image 1402, an axial temporal image 1404, a sagittal temporal image 1406, and a coronal temporal image 1408. Each CT image of the CT images 1400 includes a spatial reference point 1410 that is positioned at the same point in three-dimensional (3D) space in each CT image. The axial temporal image 1404, the sagittal temporal image 1406, and the coronal temporal image 1408 further include a ray tracing 1412 of a desired intubation tube path. The desired intubation tube path may be drawn and/or selected by a healthcare provider. One or more of the CT images 1400 may be displayed on the display 128 alongside (e.g., visually synchronized with) anesthesia data from the anesthesia system 104 and VL data from the VL system 140. For example, the CT images 1400 may be examples of patient insights 712 provided by additional imaging device(s) 134, and may be displayed as described with respect to FIG. 7. Thus, the desired intubation tube path may be visualized at the same time with real-time views of patient anatomy captured by the VL system 140, as well as patient vitals and other anesthesia data. This may increase both a speed and an accuracy of intubation.

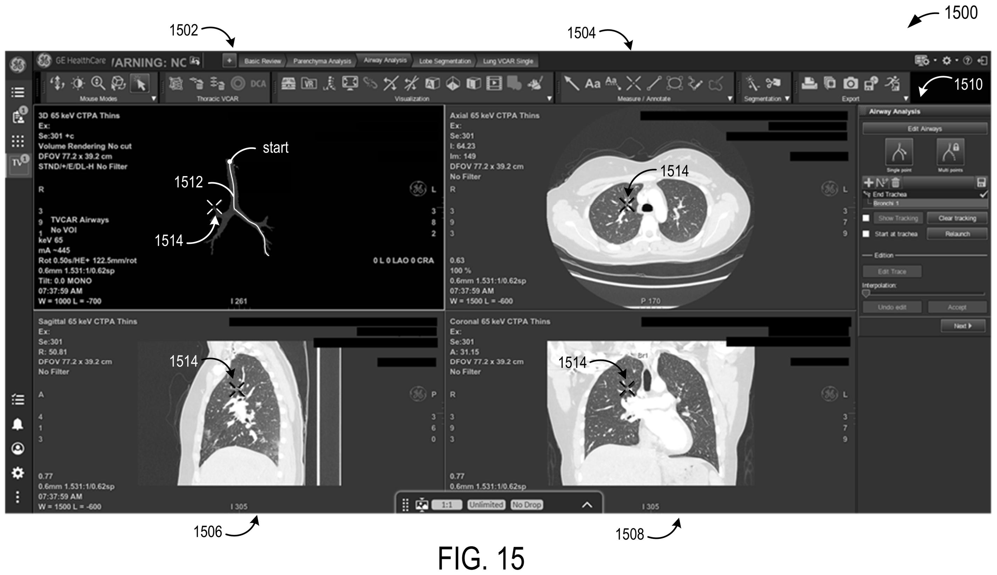

FIG. 15 shows additional patient insight images 1500, including a 3D airway image 1502, an axial image 1504, a sagittal image 1506, and a coronal image 1508. FIG. 15 further includes an airway analysis panel 1510 that enables editing of the desired intubation path via user input to the airway analysis panel (e.g., via the user input device 126 of the intubation device 102). The 3D airway image 1502 includes a ray tracing 1512 of the desired intubation tube path showing a subject airway. The axial image 1504, the sagittal image 1506, and the coronal image 1508 each include a spatial reference point 1514. The spatial reference point 1514 may be an example of a reference point described with respect to FIGS. 10-12. The 3D airway image 1502 further includes a "start" indicator. Both the "start" indicator and the spatial reference point 1514 may be automatically selected during execution of a method for determining a desired intubation tube length. In other examples, one or more of the "start" indicator and the spatial reference point 1514 may be selected by a user, such as a clinician or other healthcare provider. The desired intubation tube length may be a length between the "start" indicator and the spatial reference point 1514, for example. As described above with respect to FIG. 14, one or more of the additional patient insight images 1500 may be displayed on the display 12 alongside (e.g., visually synchronized with) anesthesia data from the anesthesia system 104 and video laryngoscopy data from the VL system 140.

FIG. 16 shows further patient insights 1600, including the 3D airway image 1502, the axial image 1504, a curved image 1602, a lumen image 1604, a second view 1606 of the 3D airway image 1502, an oblique image 1608, and the airway analysis panel 1510. The 3D airway image 1502, the curved image 1602, the lumen image 1604, and the oblique image 1608 may also include diameter measurements, which may be illustrated by a diameter outline 1610 and/or perimeter lines 1612. The diameter outline 1610 and/or perimeter lines 1612 may provide a diameter of the airway along the desired intubation tube path. This may assist in determining a size of intubation tube to use for the intubation procedure. Similar to the CT images 1400 and the additional patient insight images 1500, the further patient insights 1600 may be displayed on the display 128 alongside (e.g., visually synchronized with) anesthesia data from the anesthesia system 104 and video laryngoscopy data from the VL system 140 (e.g., the video laryngoscope 124 and/or the VL core 120 and the laryngoscope camera 122).

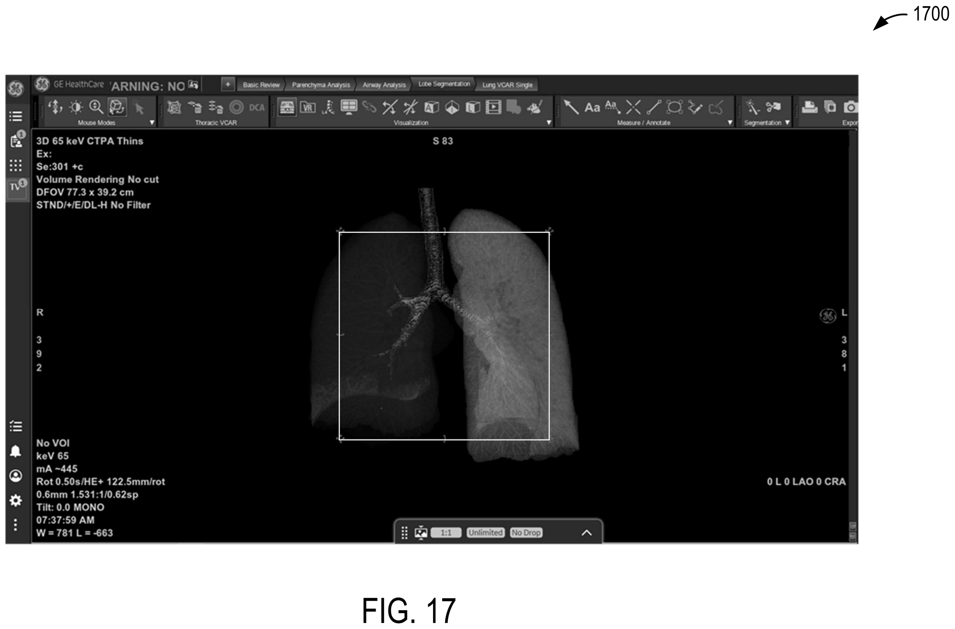

FIG. 17 shows an example 3D volume rendering 1700 of a patient anatomy. Specifically, FIG. 17 shows a volume rendering lobe segmentation example that shows a region of interest (e.g., a lung) in 3D, which may provide insight for both anesthesia and intubation.

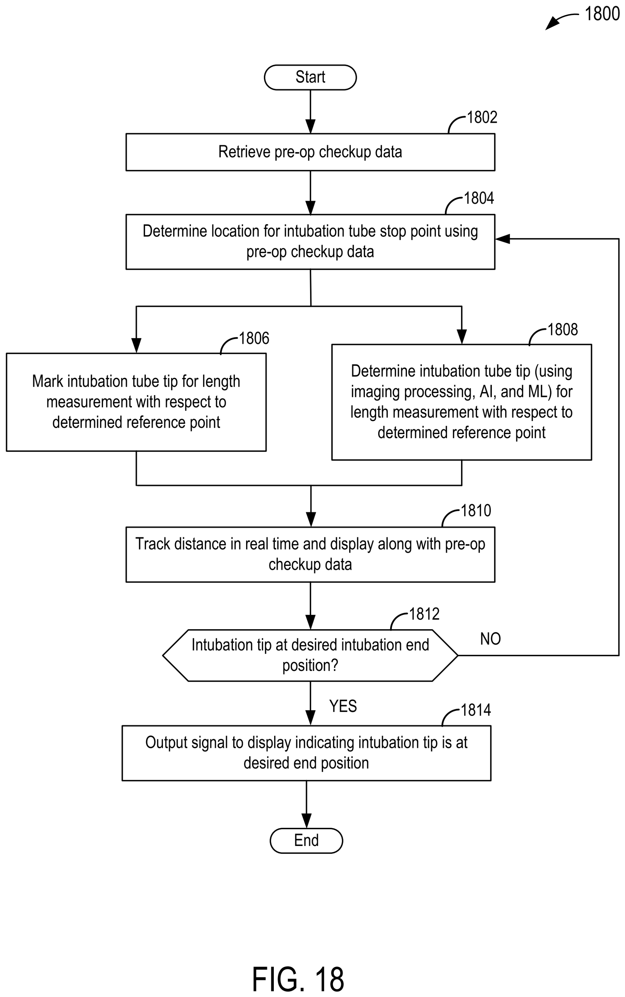

FIG. 18 shows a flow chart for a method 1800 for using imaging data to track a real-time distance between an end of an intubation tube and a desired location to place the end of the intubation tube. Imaging data may be captured in real-time via the additional imaging device(s) 134 that is communicably coupled to the intubation device 102, 600. Instructions for executing the method 1800 may be stored in the memory and executed by the processor of the intubation device 102, 600.

At 1802, the method 1800 includes retrieving pre-op checkup data. The pre-op checkup data may be retrieved by the intubation device 102, 600 (e.g., via the communication core 106) from one or more of the third-party sources 110, such as the EMR 116 and/or the PACS 118. The pre-op checkup data may include an image captured by an imaging device and/or a reference image. For example, the pre-op checkup data may include one or more of the chest x-ray 1002 of FIGS. 10-12, the CT images 1400 of FIG. 14, and the additional patient insight images 1500, 1600, and 1700 of FIGS. 15-17.

At 1804, the method 1800 includes determining a desired location for an intubation tube end point using the pre-op checkup data. The intubation tube end point may be a desired distance from the carina, as described with respect to FIGS. 10-13. The desired location for the intubation tube end point may be determined as described with respect to FIG. 13.

The method 1800 may be semi-automated and/or fully automated. In a semi-automated procedure, a user may mark an end of the intubation tube with an indicator that may be identified by the intubation device 102, 600 at 1806. For example, the user may mark the end of the intubation tube with a fluorescent marker, prior to inserting the intubation tube into the subject, where the fluorescent marker may be identified by one or more of a VL stream captured by the VL system 140 and/or a real-time image captured by the additional imaging device(s) 134 communicably coupled to and/or integrated in the intubation device 102, 600. In other examples of the semi-automated procedure, the intubation tube placement location may be extracted from the pre-op checkup data. In a fully automated procedure, the intubation tube tip may be identified automatically (e.g., by pre-trained models stored in the memory of the intubation device 102, 600) before and/or during intubation of the subject at 1808. For example, the intubation tube tip may be identified using image processing, artificial intelligence (AI), and/or a machine learning (ML) procedure. In both the semi-automated and fully automated procedures, the tip of the intubation tube may be marked to be identified for a length measurement with respect to the determined reference point (e.g., the desired location for the intubation tube end point).

At 1810, the method 1800 includes tracking a distance between the intubation tube tip and the desired intubation tube end point in real-time, and displaying the distance along with the pre-op checkup data on the display of the intubation device. The real-time position of the intubation tube tip may further by output on the display, for example, the intubation tube tip may be visible on the VL stream, and/or the marker of the intubation tube tip may be overlaid on one or more images of the pre-op checkup data.

At 1812, the method 1800 includes determining if the end of the intubation tube is at the desired intubation tube end point position. It may be determined that the intubation tube tip is at the desired intubation tube end position using at least one metric. For example, the intubation tube tip may be at the desired intubation tube end position when the distance between the intubation tube tip and the desired intubation tube end point is zero, and/or when the indicator positioned at the intubation tube tip may be visually identified as being at the same position as the reference point indicating the intubation tube stop point. If it is determined that the intubation tube tip is not at the desired end point, the method 1800 may return to 1810 to continue tracking the distance between the intubation tube tip and the desired intubation tube end point.

In response to determining that the end of the intubation tube is at the desired intubation tube end point, at 1814 the method 1800 includes outputting a signal to the display of the intubation device. The signal may be an image, text, audio, or other indicator that illustrates to a user of the intubation device 102, 600 that the intubation tip is positioned at the desired end position.

In this way, a single device is provided that integrates anesthesia and video laryngoscopy (VL) capability. The anesthesia system is an aggregator for the VL system, additional imaging device(s), sources of patient vitals to hospital network, cloud server and/or on-premises server, EMR, PACS, other image and video archiving, and so on. The anesthesia system supports integration of the VL system, sources of vitals, and additional imaging devices such as X-ray, ultrasound, CT, MRI, and so on. Integration of meaningful insights from the cloud server and/or on-premises server, with or without edge gateway, may be generated using data like pre-op checkup data, patient history, patient data, prescriptions, special conditions, clinician information, and so on.

The hospital network may be used to integrate at least the devices described herein, including the VL system, sources of patient vitals, additional imaging devices, the cloud server and/or on-premises server, and additional displays. In circumstances where some of the listed devices are communicably coupled to the hospital network and others of the listed devices are communicably coupled directly to the intubation device, the cloud server and/or the on-premises server may optionally be the integrator of all data. In environments without the cloud server and/or on-premises server, data used by the intubation device to generate insights may be directly collected by anesthesia system using medical communication standard protocols, such as HL7/IHE.

The methods and systems described herein provide clinical insights during intubation on the intubation device. This includes providing guidance during intubation with respect to human anatomy, using data taken prior to intubation along with real-time VL video stream. This may be done using static images with insights that may be retrieved from the cloud server and/or on-premises server, and a VL video live stream (e.g., displayed in real-time) with insights overlaid (e.g., layered on the static image). Guidance during intubation may also be provided with respect to human anatomy using data taken prior to intubation, real-time VL video stream, and real-time data from one or more additional imaging devices like ultrasound/X-Ray/CT/MRI, and so on. The image data from the additional imaging device(s) may be used to help track a position of the intubation tube with respect to a desired location of the intubation tube in the patient anatomy, thus providing real-time guidance. The intubation device may also generate an intubation tube length based on anatomy reference points automatically identified by the intubation device, as well as a desired intubation tube position in the patient anatomy, which may be selected by clinician and/or automatically identified by the intubation device, with or without real-time guidance (e.g., using additional imaging devices).

A user experience is also improved by implementing the methods and systems described herein. The systems and methods provide integrated Anesthesia and VL display with Anesthesia monitoring parameters, real time low latency video streaming with firewall for cyber security and parallel stream to other displays, ability to re-size/close/re-position VL screen on Anesthesia display, parallel stream with real-time and low latency to larger display, integration of Pre-Op insights on the integrated display, reduced form-factor, phase detection algorithm with complete intubation determination, and context based UI with procedure management, disabled alarms, simplified visualization of intubation phase related data, and so on.

The systems and methods described herein enable simplified record keeping. This includes storing and enabling non-complex access to evidence for future investigations and EMR/PACS and other image and video archiving integration. Recorded data can be used for training future AI models, for training clinicians, and/or for Analysis. There do not currently exist solutions on VL integrated into anesthesia devices with clinical insights including cloud/on-premises server integration and record keeping with support for additional imaging devices.

The intubation device provides specific technical solutions to technical problems in medical device integration and clinical workflow optimization. The communication core configured to send and receive data to and from a third-party source enables real-time synchronization between the anesthesia system and video laryngoscopy components through a novel architecture that reduces latency and improves reliability compared to conventional separated systems. By combining these traditionally separate medical devices into a single intubation device that includes an anesthesia system configured to deliver anesthesia to a subject and a video laryngoscopy system configured to capture a laryngoscopy video of the subject as a unified platform with shared processing and memory resources, the intubation device achieves improved video processing capabilities and more responsive clinical guidance. The technical implementation includes specific improvements through the claimed integration of data generated and/or captured by the anesthesia system and the video laryngoscopy system, and data received from the third-party source. The ability of the intubation device to process and render these multiple data streams at the same time represents a meaningful technological advance that reduces processing overhead and enables new capabilities not possible with conventional separated systems. The shared memory architecture allows for more efficient data exchange between components, reducing latency in critical clinical scenarios. The ability of the intubation device to generate intubation guidance from the combined data streams leverages the integrated nature of the intubation device to provide more accurate and responsive guidance by analyzing both video and anesthesia data streams in real-time.

These technological improvements result in concrete clinical benefits through the claimed feature of rendering data from the third-party source, the data generated and/or captured by the anesthesia system and the video laryngoscopy system, and intubation guidance at the same time on the display. This synchronized visualization enables more precise intubation tube positioning, reduced procedure times, and enhanced patient safety through coordinated monitoring of both airway visualization and anesthesia parameters. The tight integration of components through the claimed communication core enables new technical capabilities that are not achievable through mere combination of conventional separated systems.

The disclosure also provides support for an intubation device, comprising: an anesthesia system configured to deliver anesthesia to a subject, the anesthesia system comprising a communication core configured to send and receive data to and from a third-party source, a video laryngoscopy system configured to capture a laryngoscopy video of the subject, a display, a processor, and a memory storing computer-readable instructions that, when executed, cause the processor to: generate intubation guidance from data generated and/or captured by the anesthesia system and the video laryngoscopy system, and from data received from the third-party source, and render, and output for display on the display, the data received from the third-party source, the data generated and/or captured by the anesthesia system and the video laryngoscopy system, and the intubation guidance at the same time. In a first example of the system, the video laryngoscopy system comprises a video laryngoscopy core that is integrated in the anesthesia system, and a laryngoscope camera that is directly interfaced to the video laryngoscopy core via a wired and/or a wireless connection. In a second example of the system, optionally including the first example, the video laryngoscopy core is configured to generate insights about a subject airway using data captured by the laryngoscope camera and data received by the anesthesia system from the third-party source, including generating a desired length of an intubation tube and/or position of the intubation tube in the subject airway using imaging data from the third-party source and the laryngoscopy video. In a third example of the system, optionally including one or both of the first and second examples, the video laryngoscopy system comprises a video laryngoscope that is directly interfaced to the anesthesia system via a wired and/or a wireless connection with the communication core. In a fourth example of the system, optionally including one or more or each of the first through third examples, the third-party source is configured to access and/or store pre-operative checkup data, patient history, prescriptions, patient data, special conditions, clinician information, and insights generated from the pre-operative checkup data, patient history, prescriptions, patient data, special conditions, and/or clinician information. In a fifth example of the system, optionally including one or more or each of the first through fourth examples, the third-party source provides a high-speed low latency network. In a sixth example of the system, optionally including one or more or each of the first through fifth examples, the third-party source is one or more of an electronic medical records (EMR) server and a picture archiving and communication system (PACS). In a seventh example of the system, optionally including one or more or each of the first through sixth examples, the video laryngoscopy system is communicably coupled to the anesthesia system via the third-party source. In an eighth example of the system, optionally including one or more or each of the first through seventh examples, the third-party source is an edge gateway. In a ninth example of the system, optionally including one or more or each of the first through eighth examples, the third-party source is a cloud server and/or an on-premises server, and where the anesthesia system is configured to push data from the video laryngoscopy system to the cloud server and/or on-premises server, and pull data stored on the cloud server and/or on-premises server to the intubation device. In a tenth example of the system, optionally including one or more or each of the first through ninth examples, the third-party source is a hospital network. In an eleventh example of the system, optionally including one or more or each of the first through tenth examples, the system further comprises: an imaging system that is communicably coupled to the anesthesia system via the communication core. In a twelfth example of the system, optionally including one or more or each of the first through eleventh examples, the system further comprises: one or more elements for capturing subject vitals, the one or more elements communicably coupled to the anesthesia system via the communication core. In a thirteenth example of the system, optionally including one or more or each of the first through twelfth examples, intubation guidance includes indicating a desired intubation tube length of an intubation tube and a desired position of the intubation tube in a subject airway of the subject using the laryngoscopy video and the data received from the third-party source.

The disclosure also provides support for a method for an intubation device, the method comprising: administering anesthesia to a subject and monitoring anesthesia administration via an anesthesia system of an intubation device, capturing a laryngoscopy video of the subject via a video laryngoscopy system of the intubation device, sending and/or receiving subject data to and from a third-party source and a communication core of the intubation device via a high-speed, low latency network, and rendering, and outputting for display on a display of the intubation device, subject data from the third-party source alongside data generated and/or captured by the anesthesia system and the video laryngoscopy system. In a first example of the method, the method further comprises: tracking in real-time a position of an intubation tube of the intubation device in a subject anatomy of the subject using imaging data captured by an imaging device that is communicably coupled to the intubation device via the communication core, and outputting a command to halt motion of the intubation tube in response to detecting that an end of the intubation device is positioned at a desired position in the subject anatomy. In a second example of the method, optionally including the first example, the method further comprises: detecting in real-time an intubation phase of an intubation procedure using a phase detection algorithm, outputting in real-time the laryngoscopy video, managing the intubation procedure by enabling pause of anesthesia gas flow, disabling of alarms, enabling resizing of data displayed on the display, and/or visually and audibly indicating progress of intubation, and displaying anesthesia data in response to validation of complete intubation by the phase detection algorithm.

The disclosure also provides support for an intubation device, comprising: intubation administration tools including an anesthesia system and a video laryngoscopy system, a communication core that is configured as a gateway to retrieve data from one or more of an additional imaging device, a source of patient vitals, and a third-party source, a display, a processor, and a memory storing computer-readable instructions that, when executed, cause the processor to: generate intubation guidance from data generated and/or captured by the anesthesia system and the video laryngoscopy system, and from data received from the third-party source, and render and output for display on the display the data received from the third-party source, the data generated and/or captured by the anesthesia system and the video laryngoscopy system, and the intubation guidance at the same time. In a first example of the system, the video laryngoscopy system includes a video laryngoscopy core having software that is executable by the video laryngoscopy core to aggregate and process data captured by the video laryngoscopy system, data captured by the anesthesia system, and data retrieved from one or more of the additional imaging device, the source of patient vitals, and the third-party source to generate patient insights. In a second example of the system, optionally including the first example, the video laryngoscopy core is further configured to generate intubation control parameters using the patient insights, where the intubation control parameters include: detecting in real-time an intubation phase of an intubation procedure using a phase detection algorithm, outputting in real-time the laryngoscopy video, managing the intubation procedure by enabling pause of anesthesia gas flow, disabling of alarms, enabling resizing of data displayed on the display, and/or visually and audibly indicating progress of intubation, and displaying anesthesia data in response to validation of complete intubation by the phase detection algorithm.

As used herein, an element or step recited in the singular and preceded with the word "a" or "an" should be understood as not excluding plural of said elements or steps, unless such exclusion is explicitly stated. Furthermore, references to "one embodiment" of the present invention are not intended to be interpreted as excluding the existence of additional embodiments that also incorporate the recited features. Moreover, unless explicitly stated to the contrary, embodiments "comprising," "including," or "having" an element or a plurality of elements having a particular property may include additional such elements not having that property. The terms "including" and "in which" are used as the plain-language equivalents of the respective terms "comprising" and "wherein." Moreover, the terms "first," "second," and "third," etc. are used merely as labels, and are not intended to impose numerical requirements or a particular positional order on their objects.

This written description uses examples to disclose the invention, including the best mode, and also to enable a person of ordinary skill in the relevant art to practice the invention, including making and using any devices or systems and performing any incorporated methods. The patentable scope of the invention is defined by the claims, and may include other examples that occur to those of ordinary skill in the art. Such other examples are intended to be within the scope of the claims if they have structural elements that do not differ from the literal language of the claims, or if they include equivalent structural elements with insubstantial differences from the literal languages of the claims.

## Claims

1. An intubation device (102), comprising:
an anesthesia system (104) configured to deliver anesthesia to a subject and capture anesthesia data, the anesthesia system (104) comprising a communication core (106) configured to send and receive data to and from a third-party source (110);
a video laryngoscopy system (140) configured to capture a laryngoscopy video of the subject;
a display (128);
a processor (130); and
a memory (132) storing computer-readable instructions that, when executed, cause the processor (130) to:
generate intubation guidance using anesthesia data captured by the anesthesia system (104), the laryngoscopy video captured by the video laryngoscopy system (140), and data received from the third-party source (110); and
render and output for real-time display on the display (128), the intubation guidance, data received from the third-party source (110), anesthesia data, and the laryngoscopy video.

2. The intubation device (102) of claim 1, wherein the video laryngoscopy system (140) comprises a video laryngoscopy core (120) that is integrated in the anesthesia system (104), and a laryngoscope camera (122) that is directly interfaced to the video laryngoscopy core (120) via one or more of a wired connection and a wireless connection.

3. The intubation device (102) of claim 2, wherein the video laryngoscopy core (120) is configured to generate insights about a subject airway using data captured by the laryngoscope camera (122) and data received by the anesthesia system (104) from the third-party source (110), including generating one or more of a desired length of an intubation tube and a position of the intubation tube in the subject airway using imaging data from the third-party source (110) and the laryngoscopy video.

4. The intubation device (102) of claim 1, wherein the video laryngoscopy system comprises a video laryngoscope (124) that is directly interfaced to the anesthesia system (104) via one or more of a wired connection and a wireless connection with the communication core (106).

5. The intubation device (102) of claim 1, wherein the third-party source (110) is configured to access and store one or more of pre-operative checkup data, patient history, prescriptions, patient data, special conditions, clinician information, and insights generated from the pre-operative checkup data, patient history, prescriptions, patient data, special conditions, and clinician information.

6. The intubation device (102) of claim 1, wherein the third-party source (110) provides a high-speed low latency network.

7. The intubation device (102) of claim 1, wherein the third-party source (110) is one or more of an electronic medical records (EMR (116)) server and a picture archiving and communication system (PACS (118)).

8. The intubation device (102) of claim 1, wherein the video laryngoscopy system (140) is communicably coupled to the anesthesia system (104) via the third-party source (110).

9. The intubation device (102) of claim 8, wherein the third-party source (110) is an edge gateway (138).

10. The intubation device (102) of claim 8, wherein the third-party source (110) is one or more of a cloud server and an on-premises server (112), and where the anesthesia system (104) is configured to push data from the video laryngoscopy system to the cloud server and on-premises server (112), and pull data stored on the cloud server and on-premises server (112) to the intubation device (102).

11. The intubation device (102) of claim 8, wherein the third-party source (110) is a hospital network (114).

12. The intubation device (102) of claim 1, further comprising an imaging system (134) that is communicably coupled to the anesthesia system (104) via the communication core (106).

13. The intubation device (102) of claim 1, further comprising one or more elements for capturing subject vitals, the one or more elements communicably coupled to the anesthesia system (104) via the communication core (106).

14. The intubation device (102) of claim 1, wherein intubation guidance includes indicating a desired intubation tube length of an intubation tube and a desired position of the intubation tube in a subject airway of the subject using the laryngoscopy video and the data received from the third-party source (110).

15. A method (800, 900, 1300, 1800) for an intubation device (102, 600), the method (800, 900, 1300, 1800) comprising:
administering anesthesia to a subject and monitoring anesthesia administration via an anesthesia system (104) of an intubation device (102, 600);
capturing a laryngoscopy video of the subject via a video laryngoscopy system (140, 618) of the intubation device (102);
sending and receiving subject data to and from a third-party source (110) and a communication core (106) of the intubation device (102) via a high-speed, low latency network; and
rendering, and outputting for display on a display (128) of the intubation device (102, 600), subject data from the third-party source (110) alongside data acquired by the anesthesia system (104) and the video laryngoscopy system (140).
